Europäisches Patentamt

European Patent Office

Office européen des brevets

(11) Veröffentlichungsnummer: **0 397 907**
**A1**

(12) **EUROPÄISCHE PATENTANMELDUNG**

(21) Anmeldenummer: **89109038.3**

(51) Int. Cl.⁵: **A61L 2/26**

(22) Anmeldetag: **19.05.89**

(43) Veröffentlichungstag der Anmeldung:
**22.11.90 Patentblatt 90/47**

(84) Benannte Vertragsstaaten:
**AT BE CH DE ES FR GB GR IT LI LU NL**

(71) Anmelder: **Schade, Lothar**
**Sternstrasse 81**
**D-2000 Hamburg 6(DE)**

(72) Erfinder: **Schade, Lothar**
**Sternstrasse 81**
**D-2000 Hamburg 6(DE)**

(74) Vertreter: **Glaeser, Joachim, Dipl.-Ing. et al**
**Patentanwalt Königstrasse 28**
**D-2000 Hamburg 50(DE)**

(54) **Einsatz für Desinfektionsgeräte.**

(57) Auch als Aufbewahrungsbehälter verwendbares Desinfektionsgerät für Schneidmesser für Schlachtbetriebe, welches aus einem kastenförmigen Behälter (10) zur Aufnahme von Wasser, das zur Destillation über eine Heizung ausreichend erhitzt wird, aus einem oben angebrachten Schwenkdeckel (11) und aus einem mit dem Deckel in dem Behälter beweglichen Rahmen (12) mit Öffnungen (23, 24) zur Aufnahme der zu desinfizierenden Messer (60) gebildet ist, wobei je ein Hebelgestänge (13 - 18) an beiden Schmalseiten des Behälters (10) einerseits innen am Deckel (11) und andererseits mit dem Rahmen (12) verbunden ist. Das Desinfektionsgerät ist dadurch charakterisiert, daß der Rahmen (12) zwei im Abstand und parallel zueinander befestigte Platten (20, 21) aufweist, daß die Platten (20, 21) mit zueinander ausgerichteten bzw. fluchtenden Schlitzen (22, 23) zum Einstecken der Schneiden der Messer ausgebildet sind, und aus lebensmittelrechtlich unbedenklichem Kunststoff bestehen und an dem Rahmen (12) lösbar befestigt sind, daß der Rahmen (12), die Hebelgestänge (13 - 18) sowie der Behälter (10) aus Edelmetall gebildet sind, der Behälter (10) mit einem Wasserzulauf und -überlauf (51) ausgebildet ist und daß der Deckel (11) mit mindestens einem in den Behälter (10) bei zugeklapptem Deckel hineinragenden Vorsprung (30) ausgebildet ist, der so bemessen ist, daß sie die in den Schlitzen (22, 23) steckenden Schneidmesser vollständig unterhalb des Wasserspiegels (50) drücken.

Fig. 1

## Einsatz für Desinfektionsgeräte

Die Erfindung bezieht sich auf ein auch als Aufbewahrungsbehälter verwendbares Desinfektionsgerät für Schneidmesser für Schlachtbetriebe, welches aus einem kastenförmigen Behälter zur Aufnahme von Wasser, das zur Destillation über eine Heizung ausreichend erhitzt wird, aus einem oben angebrachtem Schwenkdeckel und aus einem mit dem Deckel in dem Behälter bewegbaren Rahmen mit Öffnungen zur Aufnahme der zu desinfizierenden Instrumente gebildet ist, wobei je ein Hebelgestänge an beiden Schmalseiten des Behälters einerseits innen am Deckel und andererseits mit dem Rahmen verbunden ist.

Es ist bekannt, Desinfektionsgeräte ·mit einem Einsatz zu versehen, der ähnlich wie die Einsätze in Geschirrspülmaschinen dazu dient, die zu desinfizierenden Instrumente aufzunehmen. Zu diesem Zweck ist der Einsatz mit einer Platte versehen, die Löcher aufweist, durch die Messer, Skalpelle oder dgl. eingesetzt werden können. Die bekannten Desinfektionsgeräte bzw. Einsätze arbeiten nicht zur vollsten Zufriedenheit. Zum einen ist das Unterbringen der Werkzeuge hinsichtlich der Raumausnützung nicht optimal. Zum anderen sind die Instrumente während des Desinfektionsvorganges nicht sicher genug gehalten und können Eigenbewegungen ausführen, so daß die insgesamt erzielte Desinfektionswirkung nicht zufriedenstellend ist.

Die Erfindung befaßt sich mit dem Problem, ein Desinfektionsgerät der eingangs genannten Art zu schaffen, durch das einerseits die zu desinfizierenden Schneidmesser in sicherer Lage desinfiziert werden können und durch das andererseits die Möglichkeit besteht, die Instrumente mit einem einzigen Handgriff aus dem Desinfektionsgerät zu entnehmen.

Erreicht wird dies durch einen Einsatz nach dem Patentanspruch.

Gemäß der Erfindung ist ein Hebelgestänge vorgesehen, das einen Rahmen mit dem Deckel des Desinfektionsgerätes verbindet. Der Rahmen ist in dem Desinfektionsgerät auf- und abbewegbar, so daß er in seiner oberen Lage leicht erreichbar ist, so daß die zu desinfizierenden Instrumente ohne weiteres eingebracht werden können. In seiner anderen Lage befindet sich der Rahmen im unteren Bereich des Behälters, so daß die zu desinfizierenden Instrumente sich innerhalb der Desinfektionsflüssigkeit befinden. Durch die Verbindung zwischen Rahmen und Deckel wird ermöglicht, durch Öffnen des Deckels den Rahmen in die Aufnahmestellung zu bringen und durch Schließen des Deckels den Rahmen in die Desinfektionsstellung zu bringen.

Der Rahmen selbst besteht aus mindestens zwei Etagen. Die einzelnen Etagen sind mit Schlitzen versehen, die so ausgerichtet sind, daß die Messer eingesteckt und gehaltert werden können. Soll beispielsweise ein Messer desinfiziert werden, so hat die obere Platte eine größere Öffnung und die untere Platte eine entsprechend kleinere Öffnung, so daß nur die Spitze des Messers aufgenommen werden kann, wohingegen der Schaft des Messers auf der Begrenzung der oberen Öffnung ruht. Zweckmäßigerweise sind die Platten oder Etagen des Rahmens wegnehmbar am Rahmen befestigt, so daß eine Vielfalt von unterschiedlichen Platten verwendet werden kann, um jeweils die geeignete Anpassung an die zu desinfizierenden Instrumente zu schaffen.

Die Erfindung wird nachstehend an Hand der Zeichnungen beispielsweise erläutert.

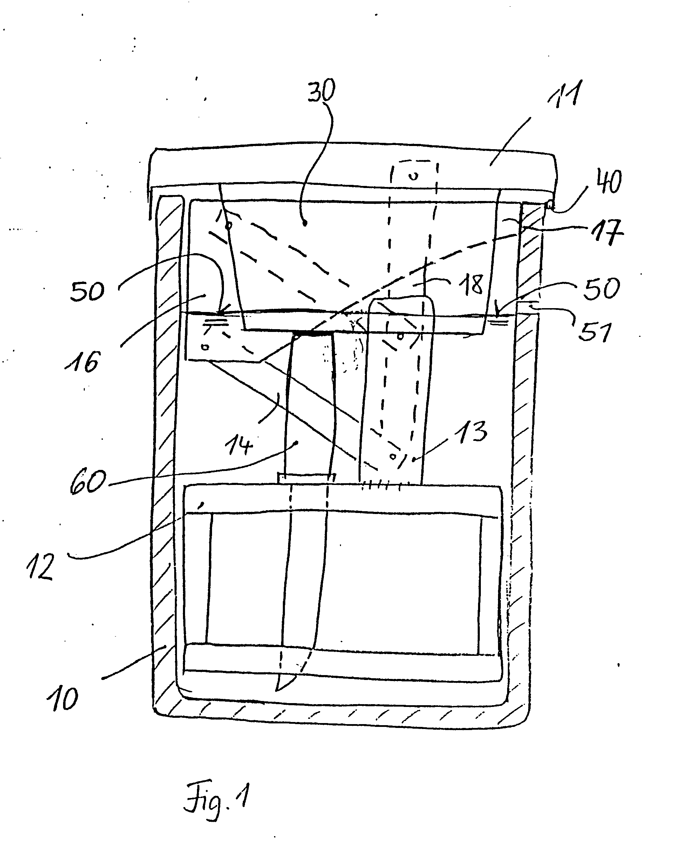

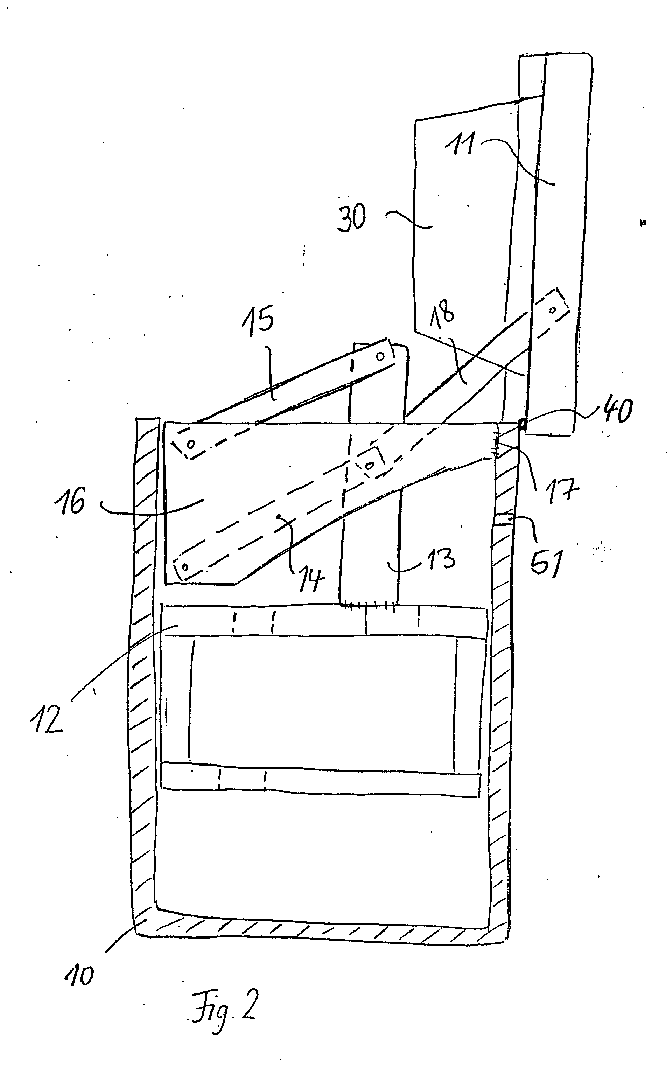

Fig. 1 und 2 zeigen in seitlicher Ansicht die Hebelgestänge eines Einsatzes gemäß der Erfindung.

Fig. 1 zeigt die Desinfektionsstellung.

Fig. 2 zeigt die Offenstellung

Fig. 3 zeigt eine schaubildliche Ansicht des Rahmens.

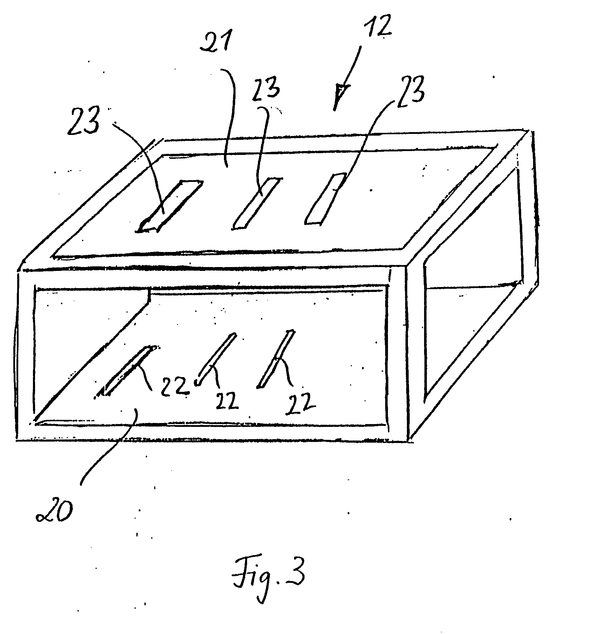

In den Fig. 1 und 2 sind die Hebelgestänge gezeigt, die einen Rahmen 12 mit einem um 90° verschwenkbaren Deckel 11 eines wannenförmigen Behälters 10 miteinander verbinden. Der wannenförmige Behälter hat im wesentlichen Quaderform, wobei zu beiden Seiten der kleinen Flächen sich ein derartiges Hebelgestänge befindet. Bei Betrachtung der Fig. 3, die lediglich den Rahmen 12 zeigt, wären diese Hebelgestänge zur linken und zur rechten Seite zu denken.

Der Behälter 10 ist nur schematisch wiedergegeben, insbesondere sind die üblichen Einrichtungen eines Desinfektionsgerätes nicht gezeigt. Selbstverständlich gehört zum Betrieb des Desinfektionsgerätes eine Vorrichtung, um die Desinfektionsflüssigkeit in den Behälter 10 zu leiten. Es gehört weiterhin eine Heizeinrichtung und möglicherweise eine Isolierung nach außen hin dazu. In den Fig. 1 und 2 ist der Behälter 10 lediglich durch eine Doppelwand wiedergegeben worden. Gezeigt ist jedoch ein Überlauf 57, der bewirkt, daß sich der Flüssigkeitsstand bei 50 einstellt.

Nachfolgend wird eines der Hebelgestänge beschrieben, wobei darauf hingewiesen wird, daß zweckmäßigerweise ein Hebelgestänge aus Flachmaterial Verwendung findet, da dies die günstigsten Platzverhältnisse ergibt. Als Materialien für den Behälter, das Gestänge aber auch den Rahmen 12, kommt ein Edelstahl oder dgl.. in Frage.

Im oberen Bereich des Behälters 10 ist bei 17 ein trapezförmiger Teil 16 befestigt, beispielsweise verschweißt. Es handelt sich um ein Blechstück, an dem zwei Gelenkteile vorgesehen sind, an denen zwei Hebel 14 und 15 gelenkig verbunden sind. Die ge genüberliegenden Enden der Hebel 14 und 15 sind an einem Blechteil 13 gelenkig angebracht, so daß der Teil 13 in bezug auf den Trapezteil 16 Parallelogrammbewegungen ausführen kann. Am unteren Ende ist das Blechteil 13 mit dem Rahmen 12 verbunden, beispielsweise verschweißt.

An dem Blechteil 13 ist noch ein weiteres Gelenk vorhanden, es kann auch das Schwenkgelenk zur Verbindung mit dem Hebel 14 hierzu verwendet werden, um einen Flachstab 18 gelenkig in bezug auf den Teil 13 zu befestigen. Das andere Ende des Stabes 18 ist mit dem Deckel 11 verbunden.

Aus den Fig. 1 und 2 ist ersichtlich, daß bei Verschwenkung des Deckels 11 über den Hebel 18 eine Auf- und Abwärtsbewegung des Teiles 13 erfolgt, so daß der Rahmen 12 über die Parallelogrammteile 14 und 15 Auf- und Abwärtsbewegungen durchführt.

Der Rahmen 12 ist schaubildlich in Fig. 3 zu erkennen. Es handelt sich hierbei um einen aus Winkelmaterial hergestellten Rahmen in Form eines Quaders. Der Rahmen weist an seinen großen Flächen oben und unten jeweils eine Platte 20 bzw. 21 auf. Diese Platte ist vorzugsweise lösbar mit dem Rahmen verbunden und besteht aus einem Kunststoff, der biologisch bzw. lebensmittelrechtlich unbedenklich ist. Sie kann beispielsweise mit Hilfe nicht gezeigter Schrauben verbunden sein, es kann jedoch auch ausreichend sein, den Rahmen und die Platten 20 und 21 über Stifte oder dgl. zu verbinden.

In der gezeigten Ausführungsform ist die obere Platte 21 mit relativ großen Schlitzen 23 versehen, wohingegen die untere Platte 20 mit fluchtenden aber wesentlich kleineren Schlitzen 22 ausgebildet ist. Es ist ersichtlich, daß mit Hilfe einer solchen Konfiguration ein Messer 60 in der oberen Platte 21 bzw. die Spitze des Messers in der unteren Platte 20 Aufnahme finden kann. Es ist auch ersichtlich, daß ein auf diese Art und Weise geführtes Messer während des Desinfektionsvorganges keine störende Bewegungen durchführen kann.

Am Deckel 1 ist ein Vorsprung 30 angebracht, der so weit in den Behälter 10 hineinvorragt, daß bei zugeklapptem Deckel 11 in den Schlitzen 23, 22 steckende Messer 60 unter die Wasseroberfläche 50 gedrückt werden und daher vollständig desinfiziert werden.

Schlachtbetriebe, welches aus einem kastenförmigen Behälter (10) zur Aufnahme von Wasser, das zur Destillation über eine Heizung ausreichend erhitzt wird, aus einem oben angebrachten Schwenkdeckel (11) und aus einem mit dem Deckel in dem Behälter bewegbaren Rahmen (12) mit Öffnungen (23, 24) zur Aufnahme der zu desinfizierenden Messer (60) gebildet ist, wobei je ein Hebelgestänge (13 - 18) an beiden Schmalseiten des Behälters (10) einerseits innen am Deckel (11) und andererseits mit dem Rahmen (12) verbunden ist, dadurch gekennzeichnet, daß der Rahmen (12) zwei im Abstand und parallel zueinander befestigte Platten (20, 21) aufweist, daß die Platten (20, 21) mit zueinander ausgerichteten bzw. fluchtenden Schlitzen (22,23) zum Einstecken der Schneiden der Messer ausgebildet sind, und aus lebensmittelrechtlich unbedenklichem Kunststoff bestehen und an dem Rahmen (12) lösbar befestigt sind, daß der Rahmen (12), die Hebelgestänge (13 - 18) sowie der Behälter (10) aus Edelmetall gebildet sind, der Behälter (10) mit einem Wasserzulauf und -überlauf (51) ausgebildet ist und daß der Deckel (11) mit mindestens einem in den Behälter (10) bei zugeklapptem Deckel hineinragenden Vorsprung (30) ausgebildet ist, der so bemessen ist, daß sie die in den Schlitzen (22, 23) steckenden Schneidmesser vollständig unterhalb des Wasserspiegels (50) drücken.

## Ansprüche

1. Auch als Aufbewahrungsbehälter verwendbares Desinfektionsgerät für Schneidmesser für

S 30927/89 - Schade

Fig. 1

S 30927/89 - Schade

Fig. 2

Fig.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-U-8 615 333 (L. SCHADE) <br> * Insgesamt * <br> --- | 1 | A 61 L 2/26 |
| X | DE-A-2 314 430 (L. SCHADE) <br> * Insgesamt * <br> --- | 1 | |
| X | US-A-3 966 408 (R. DRENNEN) <br> * Ansprüche; Figuren * <br> --- | 1 | |
| X | US-A-2 786 245 (E. STEINBOCK) <br> * Ansprüche; Figuren * <br> --- | 1 | |
| A | US-A-3 801 279 (M. GRIECO) <br> ----- | 1 | |

**RECHERCHIERTE SACHGEBIETE (Int. Cl.5)**

A 61 L
A 22 B
A 47 G
A 61 B

Der vorliegende Recherchenbericht wurde für alle Patentansprüche erstellt

| Recherchenort | Abschlußdatum der Recherche | Prüfer |
|---|---|---|
| DEN HAAG | 28-11-1989 | COUSINS-VAN STEEN G.I.L. |